# EUROPÄISCHE PATENTANMELDUNG

(11) **EP 4 696 241 A1**
(43) Veröffentlichungstag der Anmeldung: **18.02.2026**
(21) Anmeldenummer: 25187527.4
(22) Anmeldetag: 04.07.2025
(51) Int. Cl.: A61B 6/03, A61B 6/04

(54) **LAGERUNGSSTRUKTUR ZUR LAGERUNG EINES UNTERSUCHUNGSOBJEKTS EINER PÄDIATRISCHEN COMPUTERTOMOGRAPHIE-UNTERSUCHUNG UND COMPUTERTOMOGRAPHIEGERÄT**

(30) Priorität: 13.08.2024 DE 102024207709
(71) Anmelder: Siemens Healthineers AG, 91301 Forchheim (DE)
(72) Erfinder: Fehre, Jens, 91353 Hausen (DE); Ramsauer, Martin, 90602 Pyrbaum (DE)
(74) Vertreter: Siemens Healthineers Patent Attorneys

(57) **Zusammenfassung**

Die Erfindung betrifft eine Lagerungsstruktur zur Lagerung eines Untersuchungsobjekts einer pädiatrischen Computertomographie-Untersuchung,
- wobei die Lagerungsstruktur eine Auflagefläche für das Untersuchungsobjekt, eine erste Seitenwand und eine zweite Seitenwand aufweist,
- wobei sich die Auflagefläche, die erste Seitenwand und die zweite Seitenwand jeweils entlang einer Längsachse der Lagerungsstruktur flächig erstrecken,
- wobei die erste Seitenwand und die zweite Seitenwand derart relativ zu der Auflagefläche aufragen, dass das Untersuchungsobjekt zwischen der ersten Seitenwand und der zweiten Seitenwand auf die Auflagefläche gelegt werden kann.

## Beschreibung

Die Erfindung betrifft eine Lagerungsstruktur zur Lagerung eines Untersuchungsobjekts einer pädiatrischen Computertomographie-Untersuchung. Die Erfindung betrifft ferner ein Computertomographiegerät.

Bei Computertomographie-Untersuchungen ist eine genaue Ausrichtung des Untersuchungsobjekts wichtig für die Bildqualität. Bei pädiatrischen Computertomographie-Untersuchungen können dabei zusätzliche Probleme auftreten, beispielsweise in Bezug auf die Sicherheit und/oder Reinigbarkeit der Lagerung.

Die Erfindung hat die Aufgabe, eine verbesserte Schutz einer Umgebung eines Computertomographiegeräts vor einer Streustrahlung, die aus einer Öffnung einer Gantry des Computertomographiegeräts austritt, zu ermöglichen. Jeder Gegenstand eines unabhängigen Anspruchs löst diese Aufgabe. In den abhängigen Ansprüchen sind weitere vorteilhafte Aspekte der Erfindung berücksichtigt. Unabhängig vom grammatikalischen Geschlecht eines bestimmten Begriffes sind Personen mit männlicher, weiblicher oder anderer Geschlechteridentität mit umfasst.

Die Erfindung betrifft eine Lagerungsstruktur zur Lagerung eines Untersuchungsobjekts einer pädiatrischen Computertomographie-Untersuchung,
- wobei die Lagerungsstruktur eine Auflagefläche für das Untersuchungsobjekt, eine erste Seitenwand und eine zweite Seitenwand aufweist,
- wobei sich die Auflagefläche, die erste Seitenwand und die zweite Seitenwand jeweils entlang einer Längsachse der Lagerungsstruktur flächig erstrecken,
- wobei die erste Seitenwand und die zweite Seitenwand derart relativ zu der Auflagefläche aufragen, dass das Untersuchungsobjekt zwischen der ersten Seitenwand und der zweiten Seitenwand auf die Auflagefläche gelegt werden kann, insbesondere derart gelegt werden kann, dass eine longitudinale Körperachse des Untersuchungsobjekts im Wesentlichen parallel zu der Längsachse der Lagerungsstruktur ist.

Das Untersuchungsobjekt kann insbesondere ein Mensch sein. Das Untersuchungsobjekt kann beispielsweise ein Baby oder ein Kleinkind sein. Die Lagerung des Untersuchungsobjekts kann beispielsweise liegend und/oder relativ zu einer Gantry eines Computertomographiegeräts erfolgen. Das Computertomographiegerät kann insbesondere als Kopf-Computertomographiegerät für einen Kopf eines erwachsenen Menschen und/oder als mobiles Computertomographiegerät ausgebildet sein.

Insbesondere kann vorgesehen sein, dass zwischen der ersten Seitenwand und der zweiten Seitenwand eine Senke derart ausgebildet ist, dass das Untersuchungsobjekt in die Senke hinein auf die Auflagefläche gelegt werden kann. Insbesondere kann die Auflagefläche eine Sohle der Senke bilden. Insbesondere kann vorgesehen sein, dass sich die Auflagefläche quer zu der Längsachse der Lagerungsstruktur von der ersten Seitenwand bis zu der zweiten Seitenwand flächig erstreckt.

Insbesondere kann vorgesehen sein, dass die erste Seitenwand und die zweite Seitenwand derart relativ zu der Auflagefläche aufragen, dass sie durch ihr Flächenträgheitsmoment einer Verbiegung der Auflagefläche um eine Querachse der Lagerungsstruktur entgegenwirken. Insbesondere kann vorgesehen sein, dass sich die Auflagefläche entlang einer Querachse der Lagerungsstruktur von der ersten Seitenwand bis zu der zweiten Seitenwand flächig erstreckt und/oder dass die Querachse der Lagerungsstruktur zu der Längsachse der Lagerungsstruktur senkrecht ist.

Insbesondere kann vorgesehen sein, dass die Auflagefläche aus carbonfaserverstärktem Kunststoff hergestellt ist, beispielsweise derart hergestellt ist, dass eine Materialdicke der Auflagefläche kleiner als 6 mm, insbesondere kleiner als 4 mm, insbesondere nicht größer als 3 mm, ist. Insbesondere kann vorgesehen sein, dass die erste Seitenwand aus carbonfaserverstärktem Kunststoff hergestellt ist, beispielsweise derart hergestellt ist, dass eine Materialdicke der ersten Seitenwand kleiner als 6 mm, insbesondere kleiner als 4 mm, insbesondere nicht größer als 3 mm, ist. Insbesondere kann vorgesehen sein, dass die zweite Seitenwand aus carbonfaserverstärktem Kunststoff hergestellt ist, beispielsweise derart hergestellt ist, dass eine Materialdicke der zweiten Seitenwand kleiner als 6 mm, insbesondere kleiner als 4 mm, insbesondere nicht größer als 3 mm, ist.

Eine Ausführungsform sieht vor, dass in der ersten Seitenwand ein Langloch zum Einführen einer ersten Handfläche einer Bedienperson derart ausgebildet ist, dass die erste Handfläche einen an dieses Langloch grenzenden Randbereich der ersten Seitenwand umschließen kann und/oder dass in der zweiten Seitenwand ein Langloch zum Einführen einer zweiten Handfläche einer Bedienperson derart ausgebildet ist, dass die zweite Handfläche einen an dieses Langloch grenzenden Randbereich der zweiten Seitenwand umschließen kann.

Auf diese Weise können Griffe ausgebildet sein, die einen einfachen Transport sowie ein einfaches Einsetzen und/oder Entfernen der Lagerungsstruktur an dafür vorgesehenen Schnittstellen ermöglichen.

Eine Ausführungsform sieht vor, dass die Lagerungsstruktur eine erste Verbindungseinheit und eine zweite Verbindungseinheit für eine Verbindung, insbesondere für eine Klettverbindung, mit einem Haltegurt aufweist, wobei die erste Verbindungseinheit derart an der ersten Seitenwand befestigt ist und die zweite Verbindungseinheit derart an der zweiten Seitenwand befestigt ist, dass die erste Seitenwand, der Haltegurt, die zweite Seitenwand und die Auflagefläche zusammen eine umlaufend geschlossene Kontur bilden, wenn der Haltegurt mit der ersten Verbindungseinheit und mit der zweiten Verbindungseinheit verbunden ist.

Insbesondere kann vorgesehen sein, dass die erste Verbindungseinheit derart an der ersten Seitenwand befestigt ist, dass sich die erste Seitenwand zwischen der ersten Verbindungseinheit und dem Untersuchungsobjekt befindet, wenn das Untersuchungsobjekt zwischen der ersten Seitenwand und der zweiten Seitenwand auf der Auflagefläche liegt, und/oder dass die zweite Verbindungseinheit derart an der zweiten Seitenwand befestigt ist, dass sich die zweite Seitenwand zwischen der zweiten Verbindungseinheit und dem Untersuchungsobjekt befindet, wenn das Untersuchungsobjekt zwischen der ersten Seitenwand und der zweiten Seitenwand auf der Auflagefläche liegt.

Eine Ausführungsform sieht vor, dass die Lagerungsstruktur eine Rückwand aufweist, wobei die Rückwand relativ zu der Auflagefläche aufragt und sich quer zu der Längsachse der Lagerungsstruktur von der ersten Seitenwand bis zu der zweiten Seitenwand flächig erstreckt.

Insbesondere kann vorgesehen sein, dass die Rückwand derart relativ zu der Auflagefläche aufragt, dass sie durch ihr Flächenträgheitsmoment einer Verbiegung der Auflagefläche um die Längsachse der Lagerungsstruktur entgegenwirkt. Durch die Ausgestaltung der Seitenwände und der Rückwand kann somit eine ausreichende Stabilität bei möglichst geringer Materialdicke ermöglicht werden. Eine Ausführungsform sieht vor, dass in der Rückwand ein Loch zum Aufhängen der Lagerungsstruktur, insbesondere an einem Haken, ausgebildet ist.

Eine Ausführungsform sieht vor, dass die Auflagefläche, die erste Seitenwand und die zweite Seitenwand einstückig und/oder schalenförmig ineinander übergehen. Insbesondere kann vorgesehen sein, dass die Auflagefläche, die erste Seitenwand, die zweite Seitenwand und die Rückwand einstückig und/oder schalenförmig ineinander übergehen.

Dadurch können schwer reinigbare Kanten vermieden werden. Weiterhin kann vorgesehen sein, dass an dem Ende der Lagerungsstruktur, welches der Rückwand in Bezug auf die Längsachse der Lagerungsstruktur gegenüberliegt, keine relativ zu der Auflagefläche aufragende Wand vorhanden ist. Dadurch können Verunreinigungen, beispielsweise Körperflüssigkeiten des Untersuchungsobjekts, über dieses Ende von der Auflagefläche abfließen. Im Bereich der Rückwand kann die Lagerungsstruktur derart flächig geschlossen ausgebildet sein, dass dort keine flüssigen Verunreinigungen von der Auflagefläche durch die Lagerungsstruktur hindurch abfließen können, wenn die Auflagefläche im Wesentlichen horizontal angeordnet ist.

Eine Ausführungsform sieht vor, dass die Lagerungsstruktur eine Längs-Markierung aufweist, welche auf der Auflagefläche angeordnet ist und sich mittig zwischen der ersten Seitenwand und der zweiten Seitenwand entlang der Längsachse der Lagerungsstruktur erstreckt. Damit kann eine möglichst optimale Ausrichtung des Untersuchungsobjekts relativ zu der Lagerungsstruktur unterstützt werden. Die Längs-Markierung kann beispielsweise auf die Auflagefläche aufgedruckt, aufgeklebt und/oder aufgetragen sein und/oder in die Auflagefläche eingraviert sein. Die Längs-Markierung kann sich insbesondere farblich von der Lagerungsstruktur abheben. Beispielsweise kann die Längs-Markierung hell, insbesondere weiß, ausgeführt sein, wenn die Lagerungsstruktur dunkel, insbesondere schwarz, ausgeführt ist.

Die Erfindung betrifft ferner ein Computertomographiegerät, aufweisend eine Gantry mit einer Öffnung und die erfindungsgemäße Lagerungsstruktur,
- wobei sich die Öffnung derart tunnelförmig entlang einer Systemachse der Gantry erstreckt, dass die Lagerungsstruktur und das Untersuchungsobjekt zusammen entlang der Systemachse in die Öffnung einführbar sind, wenn die Längsachse der Lagerungsstruktur parallel zu der Systemachse ist und das Untersuchungsobjekt zwischen der ersten Seitenwand und der zweiten Seitenwand auf der Auflagefläche liegt,
- wobei das Computertomographiegerät eine erste geräteseitige Schnittstelle aufweist,
- wobei die Lagerungsstruktur eine lagerungsseitige Schnittstelle aufweist,
- wobei mittels der ersten geräteseitigen Schnittstelle und der lagerungsseitigen Schnittstelle eine erste lösbare Verbindung hergestellt werden kann, welche die Lagerungsstruktur derart relativ zu der Gantry fixiert, dass die Längsachse der Lagerungsstruktur parallel zu der Systemachse ist.

Insbesondere kann vorgesehen sein, dass das Computertomographiegerät einen Untersuchungstisch aufweist, wobei der Untersuchungstisch die erste geräteseitige Schnittstelle aufweist. Beispielsweise kann der Untersuchungstisch eine Basis und eine Lagerungsplatte aufweisen, wobei die Lagerungsplatte zur liegenden Lagerung eines Menschen, insbesondere eines erwachsenen Menschen, eingerichtet ist und relativ zu der Basis parallel zu der Systemachse der Gantry verschiebbar gelagert ist.

Insbesondere kann vorgesehen sein, dass die erste geräteseitige Schnittstelle an der Lagerungsplatte derart befestigt ist, dass bei einer Verschiebung der Lagerungsplatte relativ zu der Basis die erste geräteseitige Schnittstelle relativ zu der Lagerungsplatte ruht und/oder dass die Lagerungsstruktur durch die Verschiebung der Lagerungsplatte relativ zu der Basis in die Öffnung eingeführt werden kann, wenn mittels der ersten geräteseitigen Schnittstelle und der lagerungsseitigen Schnittstelle die erste lösbare Verbindung hergestellt ist, welche die Lagerungsstruktur derart relativ zu der Gantry fixiert, dass die Längsachse der Lagerungsstruktur parallel zu der Systemachse ist. Die lagerungsseitige Schnittstelle kann beispielsweise ein Aluminium-Gehäuse aufweisen.

Eine Ausführungsform sieht vor, dass die Gantry einen ersten Gantryteil und einen zweiten Gantryteil aufweist, wobei der erste Gantryteil ein Projektionsdatenakquisitionssystem aufweist, wobei der zweite Gantryteil die erste geräteseitige Schnittstelle aufweist, wobei der erste Gantryteil relativ zu dem zweiten Gantryteil derart bewegbar gelagert ist, dass eine Translationsbewegung des ersten Gantryteils relativ zu dem zweiten Gantryteil parallel zu der Systemachse ausgeführt werden kann, während gleichzeitig dazu die Lagerungsstruktur relativ zu dem zweiten Gantryteil ruht, wobei mittels der ersten geräteseitigen Schnittstelle und der lagerungsseitigen Schnittstelle die erste lösbare Verbindung hergestellt ist, welche die Lagerungsstruktur derart relativ zu der Gantry fixiert, dass die Längsachse der Lagerungsstruktur parallel zu der Systemachse ist.

Eine Ausführungsform sieht vor, dass die Lagerungsstruktur eine erste Quer-Markierung und eine zweite Quer-Markierung aufweist, welche jeweils auf der Auflagefläche angeordnet sind und sich senkrecht zu der Längsachse der Lagerungsstruktur erstrecken, wobei die erste Quer-Markierung und die zweite Quer-Markierung eine Erstreckung eines Untersuchungsbereichs, der durch die Translationsbewegung des ersten Gantryteils relativ zu dem zweiten Gantryteil für das Projektionsdatenakquisitionssystem erreichbar ist, entlang der Systemachse markieren.

Damit kann eine möglichst optimale Ausrichtung des Untersuchungsobjekts relativ zu der Lagerungsstruktur unterstützt werden. Die Quer-Markierungen können beispielsweise auf die Auflagefläche aufgedruckt, aufgeklebt und/oder aufgetragen sein und/oder in die Auflagefläche eingraviert sein. Die Quer-Markierungen können sich insbesondere farblich von der Lagerungsstruktur abheben. Beispielsweise können die Quer-Markierungen hell, insbesondere weiß, ausgeführt sein, wenn die Lagerungsstruktur dunkel, insbesondere schwarz, ausgeführt ist.

Eine Ausführungsform sieht vor, dass der zweite Gantryteil eine zweite geräteseitige Schnittstelle aufweist, wobei mittels der zweiten geräteseitigen Schnittstelle und der lagerungsseitigen Schnittstelle eine zweite lösbare Verbindung hergestellt werden kann, welche die Lagerungsstruktur derart relativ zu der Gantry fixiert, dass die Längsachse der Lagerungsstruktur senkrecht und windschief zu der Systemachse ist. Eine Ausführungsform sieht vor, dass der zweite Gantryteil ein Fahrwerk aufweist, wobei das Fahrwerk zum Bewegen der Gantry relativ zu einem Boden eines Untersuchungsraumes parallel zu einer Translationsrichtung eingerichtet ist, wobei die Translationsrichtung senkrecht zu der Systemachse ist.

Insbesondere kann vorgesehen sein, dass die Systemachse horizontal ist und/oder dass die Translationsrichtung horizontal ist. Insbesondere kann vorgesehen sein, dass die Längsachse der Lagerungsstruktur im Wesentlichen parallel zu der Translationsrichtung ist, wenn mittels der zweiten geräteseitigen Schnittstelle und der lagerungsseitigen Schnittstelle die zweite lösbare Verbindung hergestellt ist, welche die Lagerungsstruktur derart relativ zu der Gantry fixiert, dass die Längsachse der Lagerungsstruktur senkrecht und windschief zu der Systemachse ist. Dadurch kann für das Bewegen der Gantry relativ zu dem Boden des Untersuchungsraumes ein Platzbedarf für das Computertomographiegerät quer zu der Translationsrichtung verringert werden.

Eine Ausführungsform sieht vor, dass das Computertomographiegerät eine Haltestruktur für die Lagerungsstruktur aufweist, wobei eine erste Teilstruktur der Haltestruktur die zweite geräteseitige Schnittstelle aufweist, wobei der zweite Gantryteil eine Mulde zur formschlüssigen Aufnahme der Haltestruktur derart aufweist, dass die erste Teilstruktur der Haltestruktur aus der Mulde herausragt, wenn die Haltestruktur in die Mulde formschlüssig aufgenommen ist.

Insbesondere kann vorgesehen sein, dass eine Kontaktfläche der zweiten Teilstruktur der Haltestruktur an einem Boden der Mulde anliegt, wenn die Haltestruktur in die Mulde formschlüssig aufgenommen ist. Die Haltestruktur kann beispielsweise aus Kunststoff, insbesondere aus einem Thermoplast, bevorzugt aus Polyoxymethylen (POM), hergestellt sein.

Eine Ausführungsform sieht vor, dass eine zweite Teilstruktur der Haltestruktur eine tischseitige Schnittstelle aufweist, wobei mittels der tischseitigen Schnittstelle und der lagerungsseitigen Schnittstelle eine dritte lösbare Verbindung hergestellt werden kann, welche die Lagerungsstruktur derart relativ zu der Haltestruktur fixiert, dass die Längsachse der Lagerungsstruktur im Wesentlichen parallel zu einer Tischplatte ist, wenn die Haltestruktur derart auf der Tischplatte abgestellt ist, dass eine Kontaktfläche der ersten Teilstruktur der Haltestruktur an der Tischplatte anliegt. Die Kontaktfläche der ersten Teilstruktur der Haltestruktur und die Kontaktfläche der zweiten Teilstruktur der Haltestruktur können insbesondere planparallel sein.

Eine Ausführungsform sieht vor, dass die Haltestruktur einen ersten Schenkelbereich, einen zweiten Schenkelbereich und einen Scheitelbereich aufweist, wobei der Scheitelbereich die zweite geräteseitige Schnittstelle und/oder die tischseitige Schnittstelle aufweist und sich von dem ersten Schenkelbereich bis zu dem zweiten Schenkelbereich erstreckt, wobei sich der erste Schenkelbereich und der zweite Schenkelbereich parallel zu der Längsachse der Lagerungsstruktur weg von dem Scheitelbereich erstrecken, wenn die zweite lösbare Verbindung oder die dritte lösbare Verbindung hergestellt ist.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung der unbestimmten Artikel "ein" bzw. "eine" schließt nicht aus, dass das betroffene Merkmal auch mehrfach vorhanden sein kann.

Im Folgenden werden Merkmale der Erfindung anhand von Beispielen unter Hinweis auf die beigefügten Figuren erläutert. Die Darstellung in den Figuren ist schematisch, stark vereinfacht und nicht zwingend maßstabsgetreu.

Die Fig. 1 zeigt ein mobiles Computertomographiegerät.

Die Fig. 2 zeigt ein Computertomographiegerät mit einem Untersuchungstisch.

Die Fig. 3 bis Fig. 6 zeigen verschiedene Ansichten einer Lagerungsstruktur.

Die Fig. 7 zeigt ein mobiles Computertomographiegerät in einer weiteren Ansicht.

Die Fig. 8 zeigt eine Mulde zur formschlüssigen Aufnahme der Haltestruktur.

Die Fig. 9 zeigt eine Haltestruktur in einer ersten Orientierung.

Die Fig. 10 zeigt eine Anordnung einer Lagerungsstruktur an einer Gantry.

Die Fig. 11 zeigt eine Haltestruktur in einer zweiten Orientierung.

Die Fig. 12 zeigt eine Anordnung einer Lagerungsstruktur auf einer Tischplatte.

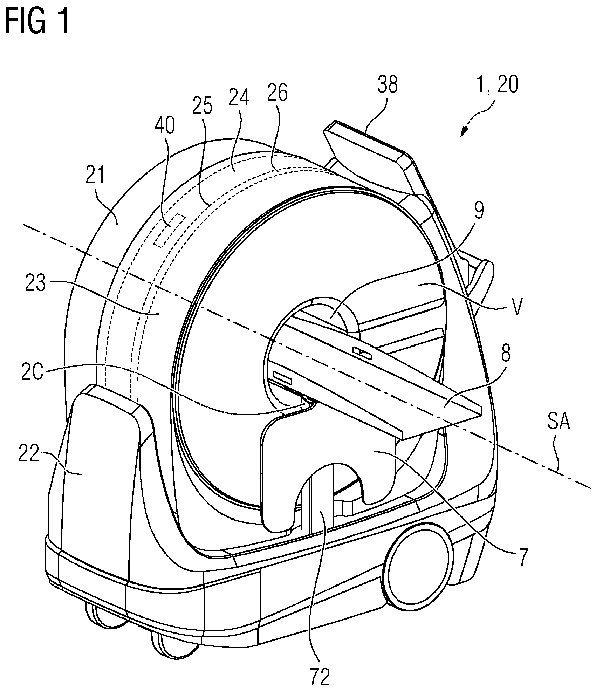

Die Fig. 1 zeigt das Computertomographiegerät 1 in Form eines mobilen Computertomographiegeräts, aufweisend eine Gantry 20 mit einer Öffnung 9 und die Lagerungsstruktur 8,
- wobei sich die Öffnung 9 derart tunnelförmig entlang einer Systemachse SA der Gantry 20 erstreckt, dass die Lagerungsstruktur 8 und das Untersuchungsobjekt 13 zusammen entlang der Systemachse SA in die Öffnung 9 einführbar sind, wenn die Längsachse der Lagerungsstruktur 8 parallel zu der Systemachse SA ist und das Untersuchungsobjekt 13 zwischen der ersten Seitenwand 81 und der zweiten Seitenwand 82 auf der Auflagefläche 80 liegt,
- wobei das Computertomographiegerät 1 eine erste geräteseitige Schnittstelle 1C, 2C aufweist,
- wobei die Lagerungsstruktur 8 eine lagerungsseitige Schnittstelle C aufweist,
- wobei mittels der ersten geräteseitigen Schnittstelle 1C, 2C und der lagerungsseitigen Schnittstelle C eine erste lösbare Verbindung hergestellt werden kann, welche die Lagerungsstruktur 8 derart relativ zu der Gantry 20 fixiert, dass die Längsachse der Lagerungsstruktur 8 parallel zu der Systemachse SA ist.

Das gezeigte Beispiel sieht vor, dass die Gantry 20 einen ersten Gantryteil 21 und einen zweiten Gantryteil 22 aufweist,
- wobei der erste Gantryteil 21 ein Projektionsdatenakquisitionssystem 40 aufweist,
- wobei der zweite Gantryteil 22 die erste geräteseitige Schnittstelle 2C aufweist,
- wobei der erste Gantryteil 21 relativ zu dem zweiten Gantryteil 22 derart bewegbar gelagert ist, dass eine Translationsbewegung des ersten Gantryteils 21 relativ zu dem zweiten Gantryteil 22 parallel zu der Systemachse SA ausgeführt werden kann, während gleichzeitig dazu die Lagerungsstruktur 8 relativ zu dem zweiten Gantryteil 22 ruht, wobei mittels der ersten geräteseitigen Schnittstelle 1C, 2C und der lagerungsseitigen Schnittstelle C die erste lösbare Verbindung hergestellt ist, welche die Lagerungsstruktur 8 derart relativ zu der Gantry 20 fixiert, dass die Längsachse der Lagerungsstruktur 8 parallel zu der Systemachse SA ist.

Der erste Gantryteil 21 weist die Drehlagerung 25 und die Tragstruktur 26 auf, wobei der Rotor 24 mittels der Drehlagerung 25 mit der Tragstruktur 26 verbunden und relativ zu der Tragstruktur 26 um die Systemachse SA drehbar gelagert ist. Der Rotor 24 weist das Projektionsdatenakquisitionssystem 40 auf. Das Projektionsdatenakquisitionssystem 40 weist die Röntgenquelle 41 zum Erzeugen der Röntgenstrahlung 4 und den Röntgendetektor 44 zum Detektieren der Röntgenstrahlung 4 auf.

Der zweite Gantryteil 22 weist die Haltevorrichtung 7, das Schulterbrett 7 und den berührungsempfindlichen Bildschirm 38 zur Bedienung des Computertomographiegeräts 1 auf. Das Schulterbrett 7 ist mittels einer Schwenkvorrichtung mit der Haltevorrichtung 7 verbunden und relativ zu der Haltevorrichtung 7 um eine Schwenkachse, die horizontal und zu der Systemachse SA senkrecht ist, schwenkbar gelagert.

Die Gantry 20 weist die Verkleidung V zur Abgrenzung eines Innenbereichs der Gantry 20 von einer Umgebung der Gantry 20 auf. Die Gantry 20 weist den dritten Gantryteil 23 auf. Der dritte Gantryteil 23 weist eine Vorderseite der Verkleidung V auf. Die Vorderseite der Verkleidung V umgibt ringförmig eine Vorderseite der Öffnung 9. Der erste Gantryteil 21 weist eine Rückseite der Verkleidung V auf. Die Rückseite der Verkleidung V umgibt ringförmig eine Rückseite der Öffnung 9.

Der erste Gantryteil 21 ist relativ zu dem zweiten Gantryteil 22 und relativ zu dem dritten Gantryteil 23 derart bewegbar gelagert, dass die Translationsbewegung des ersten Gantryteils 21 relativ zu dem zweiten Gantryteil 22 und dem dritten Gantryteil 23 ausgeführt werden kann, während gleichzeitig dazu der zweite Gantryteil 22 und der dritte Gantryteil 23 relativ zu der Lagerungsstruktur 8 ruhen, wobei die Lagerungsstruktur 8 zusammen mit dem Untersuchungsobjekt 13 in die Öffnung 9 eingeführt ist.

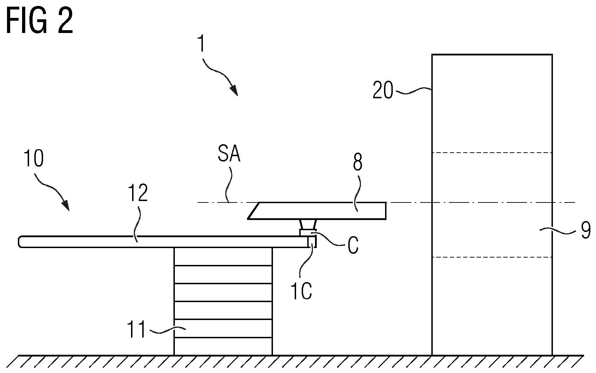

Die Fig. 2 zeigt das Computertomographiegerät 1 mit dem Untersuchungstisch 10, wobei der Untersuchungstisch 10 die erste geräteseitige Schnittstelle 1C aufweist. Beispielsweise kann der Untersuchungstisch 10 eine Basis 11 und eine Lagerungsplatte 12 aufweisen, wobei die Lagerungsplatte 12 zur liegenden Lagerung eines Menschen, insbesondere eines erwachsenen Menschen, eingerichtet ist und relativ zu der Basis 11 parallel zu der Systemachse SA der Gantry 20 verschiebbar gelagert ist.

Insbesondere kann vorgesehen sein, dass die erste geräteseitige Schnittstelle 1C an der Lagerungsplatte 12 derart befestigt ist, dass bei einer Verschiebung der Lagerungsplatte 12 relativ zu der Basis 11 die erste geräteseitige Schnittstelle 1C relativ zu der Lagerungsplatte 12 ruht und/oder dass die Lagerungsstruktur 8 durch die Verschiebung der Lagerungsplatte 12 relativ zu der Basis 11 in die Öffnung 9 eingeführt werden kann, wenn mittels der ersten geräteseitigen Schnittstelle 1C und der lagerungsseitigen Schnittstelle C die erste lösbare Verbindung hergestellt ist, welche die Lagerungsstruktur 8 derart relativ zu der Gantry 20 fixiert, dass die Längsachse der Lagerungsstruktur 8 parallel zu der Systemachse SA ist.

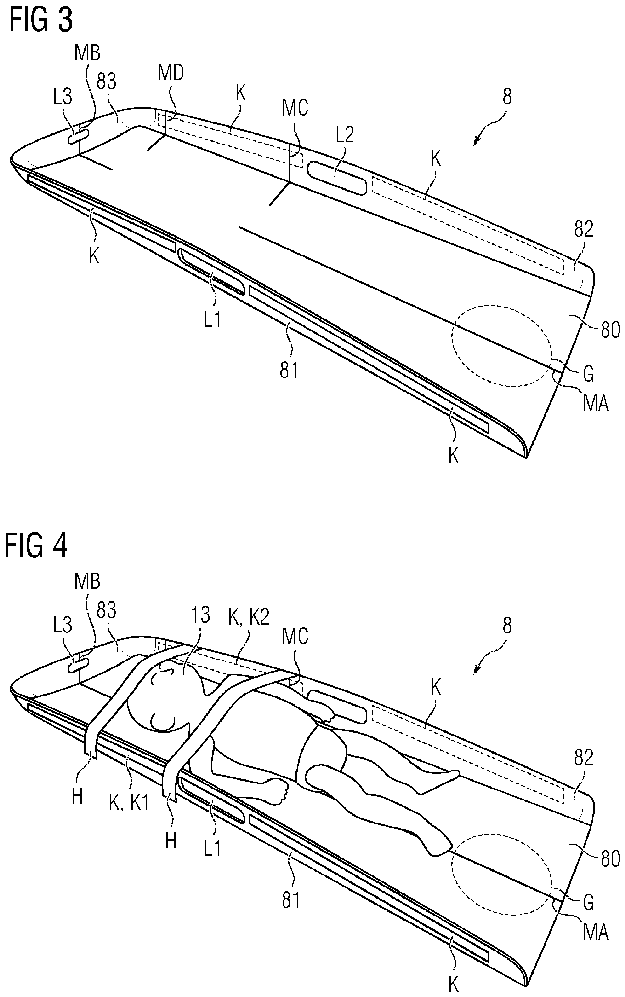

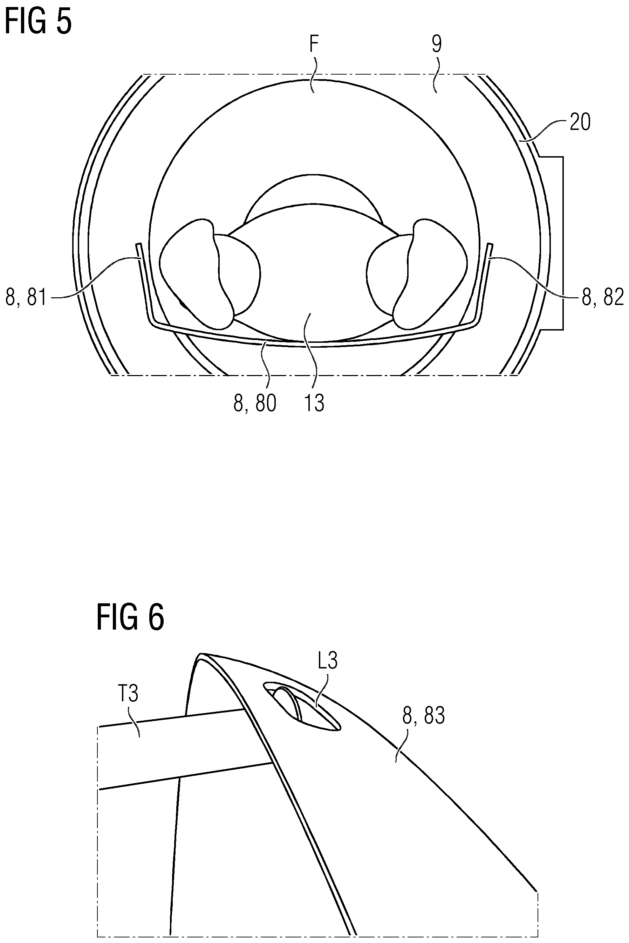

Die Fig. 3 bis Fig. 6 zeigen verschiedene Ansichten der Lagerungsstruktur 8 zur Lagerung des Untersuchungsobjekts 13 einer pädiatrischen Computertomographie-Untersuchung,
- wobei die Lagerungsstruktur 8 eine Auflagefläche 80 für das Untersuchungsobjekt 13, eine erste Seitenwand 81 und eine zweite Seitenwand 82 aufweist,
- wobei sich die Auflagefläche 80, die erste Seitenwand 81 und die zweite Seitenwand 82 jeweils entlang einer Längsachse der Lagerungsstruktur 8 flächig erstrecken,
- wobei die erste Seitenwand 81 und die zweite Seitenwand 82 derart relativ zu der Auflagefläche 80 aufragen, dass das Untersuchungsobjekt 13 zwischen der ersten Seitenwand 81 und der zweiten Seitenwand 82 auf die Auflagefläche 80 gelegt werden kann.

Insbesondere kann vorgesehen sein, dass die erste Seitenwand 81 und die zweite Seitenwand 82 derart relativ zu der Auflagefläche 80 aufragen, dass sie durch ihr Flächenträgheitsmoment einer Verbiegung der Auflagefläche 80 um eine Querachse der Lagerungsstruktur 8 entgegenwirken. Insbesondere kann vorgesehen sein, dass sich die Auflagefläche 80 entlang einer Querachse der Lagerungsstruktur 8 von der ersten Seitenwand 81 bis zu der zweiten Seitenwand 82 flächig erstreckt und/oder dass die Querachse der Lagerungsstruktur 8 zu der Längsachse der Lagerungsstruktur 8 senkrecht ist.

Das gezeigte Beispiel sieht vor, dass in der ersten Seitenwand 81 ein Langloch L1 zum Einführen einer ersten Handfläche einer Bedienperson derart ausgebildet ist, dass die erste Handfläche einen an dieses Langloch L1 grenzenden Randbereich der ersten Seitenwand 81 umschließen kann und/oder
- wobei in der zweiten Seitenwand 82 ein Langloch L2 zum Einführen einer zweiten Handfläche einer Bedienperson derart ausgebildet ist, dass die zweite Handfläche einen an dieses Langloch L2 grenzenden Randbereich der zweiten Seitenwand 82 umschließen kann.

Das gezeigte Beispiel sieht vor, dass die Lagerungsstruktur 8 eine erste Verbindungseinheit K1 und eine zweite Verbindungseinheit K2 für eine Verbindung mit einem Haltegurt H aufweist,
- wobei die erste Verbindungseinheit K1 derart an der ersten Seitenwand 81 befestigt ist und die zweite Verbindungseinheit K2 derart an der zweiten Seitenwand 82 befestigt ist, dass die erste Seitenwand 81, der Haltegurt H, die zweite Seitenwand 82 und die Auflagefläche 80 zusammen eine umlaufend geschlossene Kontur bilden, wenn der Haltegurt H mit der ersten Verbindungseinheit K1 und mit der zweiten Verbindungseinheit K2 verbunden ist.

Insbesondere kann vorgesehen sein, dass die erste Verbindungseinheit K1 derart an der ersten Seitenwand 81 befestigt ist, dass sich die erste Seitenwand 81 zwischen der ersten Verbindungseinheit K1 und dem Untersuchungsobjekt 13 befindet, wenn das Untersuchungsobjekt 13 zwischen der ersten Seitenwand 81 und der zweiten Seitenwand 82 auf der Auflagefläche 80 liegt, und/oder dass die zweite Verbindungseinheit K2 derart an der zweiten Seitenwand 82 befestigt ist, dass sich die zweite Seitenwand 82 zwischen der zweiten Verbindungseinheit K2 und dem Untersuchungsobjekt 13 befindet, wenn das Untersuchungsobjekt 13 zwischen der ersten Seitenwand 81 und der zweiten Seitenwand 82 auf der Auflagefläche 80 liegt.

Das gezeigte Beispiel sieht vor, dass die Lagerungsstruktur 8 eine Rückwand 83 aufweist, wobei die Rückwand 83 relativ zu der Auflagefläche 80 aufragt und sich quer zu der Längsachse der Lagerungsstruktur 8 von der ersten Seitenwand 81 bis zu der zweiten Seitenwand 82 flächig erstreckt. Insbesondere kann vorgesehen sein, dass die Rückwand 83 derart relativ zu der Auflagefläche 80 aufragt, dass sie durch ihr Flächenträgheitsmoment einer Verbiegung der Auflagefläche 80 um die Längsachse der Lagerungsstruktur 8 entgegenwirkt.

Das gezeigte Beispiel sieht vor, dass in der Rückwand 83 ein Loch L3 zum Aufhängen der Lagerungsstruktur 8, insbesondere an einem Haken T3, ausgebildet ist. Das gezeigte Beispiel sieht vor, dass die Auflagefläche 80, die erste Seitenwand 81 und die zweite Seitenwand 82 einstückig und/oder schalenförmig ineinander übergehen. Insbesondere kann vorgesehen sein, dass die Auflagefläche 80, die erste Seitenwand 81, die zweite Seitenwand 82 und die Rückwand 83 einstückig und/oder schalenförmig ineinander übergehen. Das gezeigte Beispiel sieht vor, dass die Lagerungsstruktur 8 eine Längs-Markierung MA aufweist, welche auf der Auflagefläche 80 angeordnet ist und sich mittig zwischen der ersten Seitenwand 81 und der zweiten Seitenwand 82 entlang der Längsachse der Lagerungsstruktur 8 erstreckt.

Das gezeigte Beispiel sieht vor, dass die Lagerungsstruktur 8 eine erste Quer-Markierung MC und eine zweite Quer-Markierung MD aufweist, welche jeweils auf der Auflagefläche 80 angeordnet sind und sich senkrecht zu der Längsachse der Lagerungsstruktur 8 erstrecken,
- wobei die erste Quer-Markierung MC und die zweite Quer-Markierung MD eine Erstreckung eines Untersuchungsbereichs, der durch die Translationsbewegung des ersten Gantryteils 21 relativ zu dem zweiten Gantryteil 22 für das Projektionsdatenakquisitionssystem 40 erreichbar ist, entlang der Systemachse SA markieren.

An den Seitenwänden sind Klettbänder K befestigt, welche die erste Verbindungseinheit K1 und die zweite Verbindungseinheit K2 für eine Klettverbindung mit dem Haltegurt H bilden.

Die Auflagefläche 80 weist eine Ablagefläche G für medizintechnisches Zubehör auf, das beispielsweise mit dem Untersuchungsobjekt 13 verbunden und/oder mittels eines Haltegurts relativ zu der Lagerungsstruktur 8 fixiert sein kann. Die Zentral-Markierung MB an der Rückwand 83 erstreckt sich mittig zwischen der ersten Seitenwand 81 und der zweiten Seitenwand 82 und ist komplanar zu der Längs-Markierung MA.

Die Lagerungsstruktur 8 ist derart geformt, dass die erste Seitenwand 81 und die zweite Seitenwand 82 sich außerhalb des Akquisitionsbereichs F des

Projektionsdatenakquisitionssystems 40 befinden. Der Akquisitionsbereich F kann als Field of View verstanden werden. Dadurch können die Bildgebung störende Kanten vermieden werden. Die Auflagefläche 80 ist derart flach und stabil ausgeformt, dass sie auch bei Belastung derart relativ zu dem Akquisitionsbereich F angeordnet bleibt, dass sich aus ihr keine die Bildgebung störenden Kanten ergeben.

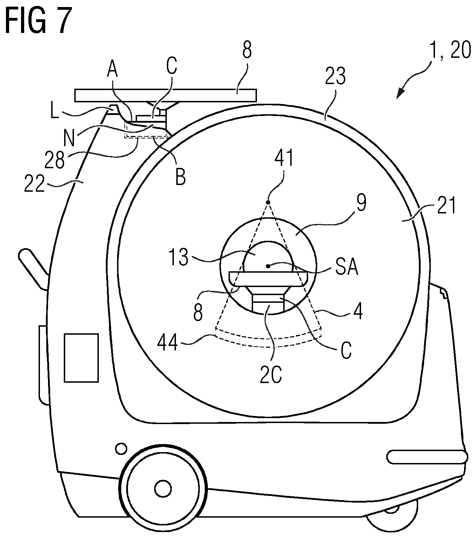

Die Fig. 7 zeigt das Computertomographiegerät 1 in Form eines mobilen Computertomographiegeräts in einer weiteren Ansicht. Das gezeigte Beispiel sieht vor, dass der zweite Gantryteil 22 eine zweite geräteseitige Schnittstelle AC aufweist,
- wobei mittels der zweiten geräteseitigen Schnittstelle AC und der lagerungsseitigen Schnittstelle C eine zweite lösbare Verbindung hergestellt werden kann, welche die Lagerungsstruktur 8 derart relativ zu der Gantry 20 fixiert, dass die Längsachse der Lagerungsstruktur 8 senkrecht und windschief zu der Systemachse SA ist.

Das gezeigte Beispiel sieht vor, dass der zweite Gantryteil 22 ein Fahrwerk aufweist, wobei das Fahrwerk zum Bewegen der Gantry 20 relativ zu einem Boden eines Untersuchungsraumes parallel zu einer Translationsrichtung eingerichtet ist, wobei die Translationsrichtung senkrecht zu der Systemachse SA ist. Insbesondere kann vorgesehen sein, dass die Systemachse SA horizontal ist und/oder dass die Translationsrichtung horizontal ist. Insbesondere kann vorgesehen sein, dass die Längsachse der Lagerungsstruktur 8 im Wesentlichen parallel zu der Translationsrichtung ist, wenn mittels der zweiten geräteseitigen Schnittstelle AC und der lagerungsseitigen Schnittstelle C die zweite lösbare Verbindung hergestellt ist, welche die Lagerungsstruktur 8 derart relativ zu der Gantry 20 fixiert, dass die Längsachse der Lagerungsstruktur 8 senkrecht und windschief zu der Systemachse SA ist.

Das gezeigte Beispiel sieht vor, dass das Computertomographiegerät 1 eine Haltestruktur N für die Lagerungsstruktur 8 aufweist,
- wobei eine erste Teilstruktur A der Haltestruktur N die zweite geräteseitige Schnittstelle AC aufweist,
- wobei der zweite Gantryteil 22 eine Mulde 28 zur formschlüssigen Aufnahme der Haltestruktur N derart aufweist, dass die erste Teilstruktur A der Haltestruktur N aus der Mulde 28 herausragt, wenn die Haltestruktur N in die Mulde 28 formschlüssig aufgenommen ist.

Insbesondere kann vorgesehen sein, dass eine Kontaktfläche der zweiten Teilstruktur BC der Haltestruktur N an einem Boden der Mulde 28 anliegt, wenn die Haltestruktur N in die Mulde 28 formschlüssig aufgenommen ist.

Die Mulde 28 ist unterhalb der Warnlampe L ausgebildet.

In der Fig. 7 ist das Computertomographiegerät 1 mit zwei Lagerungsstrukturen 8 gezeigt. Auf einer der beiden Lagerungsstrukturen 8 ist das Untersuchungsobjekt 13 für eine Untersuchung mittels des Computertomographiegeräts 1 gelagert.

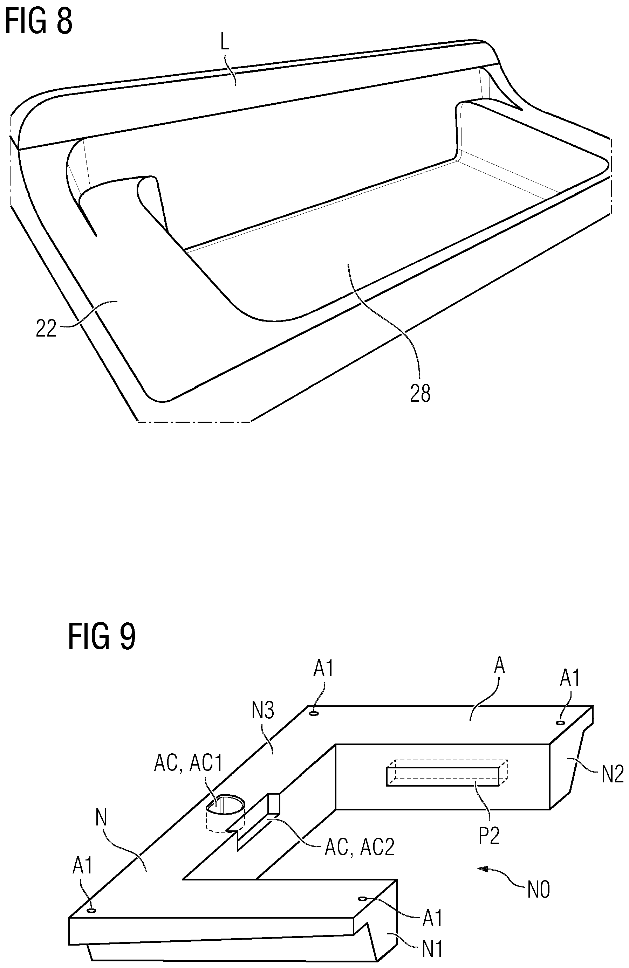

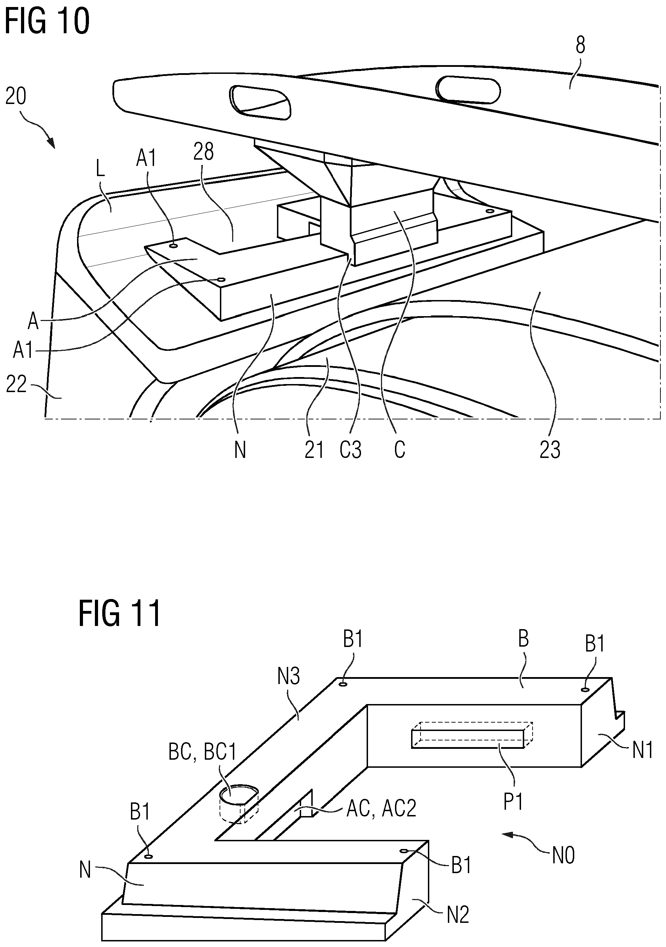

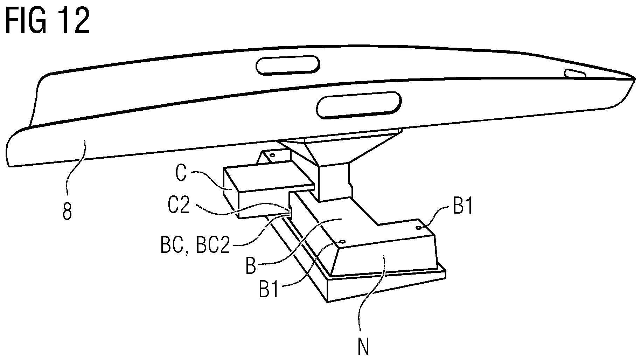

Die Fig. 8 zeigt die Mulde 28 zur formschlüssigen Aufnahme der Haltestruktur N. Die Fig. 9 zeigt die Haltestruktur N in einer ersten Orientierung. Die Fig. 10 zeigt eine Anordnung der Lagerungsstruktur 8 an der Gantry 20 mit Hilfe der Haltestruktur N. Die Fig. 11 zeigt die Haltestruktur N in einer zweiten Orientierung. Die Fig. 12 zeigt eine Anordnung der Lagerungsstruktur 8 auf einer Tischplatte mit Hilfe der Haltestruktur N.

Das gezeigte Beispiel sieht vor, dass eine zweite Teilstruktur B der Haltestruktur N eine tischseitige Schnittstelle BC aufweist,
- wobei mittels der tischseitigen Schnittstelle BC und der lagerungsseitigen Schnittstelle C eine dritte lösbare Verbindung hergestellt werden kann, welche die Lagerungsstruktur 8 derart relativ zu der Haltestruktur N fixiert, dass die Längsachse der Lagerungsstruktur 8 im Wesentlichen parallel zu einer Tischplatte ist, wenn die Haltestruktur N derart auf der Tischplatte abgestellt ist, dass eine Kontaktfläche der ersten Teilstruktur A der Haltestruktur N an der Tischplatte anliegt. Die Kontaktfläche der ersten Teilstruktur A der Haltestruktur N und die Kontaktfläche der zweiten Teilstruktur B der Haltestruktur N können insbesondere planparallel sein.

Das gezeigte Beispiel sieht vor, dass die Haltestruktur N einen ersten Schenkelbereich N1, einen zweiten Schenkelbereich N2 und einen Scheitelbereich N3 aufweist,
- wobei der Scheitelbereich N3 die zweite geräteseitige Schnittstelle AC und/oder die tischseitige Schnittstelle BC aufweist und sich von dem ersten Schenkelbereich N1 bis zu dem zweiten Schenkelbereich N2 erstreckt,
- wobei sich der erste Schenkelbereich N1 und der zweite Schenkelbereich N2 parallel zu der Längsachse der Lagerungsstruktur 8 weg von dem Scheitelbereich N3 erstrecken, wenn die zweite lösbare Verbindung oder die dritte lösbare Verbindung hergestellt ist.

Die Haltestruktur N ist U-förmig ausgebildet. Die Haltestruktur N weist zwischen dem ersten Schenkelbereich N1 und dem zweiten Schenkelbereich N2 die Aussparung N0 auf. Der erste Schenkelbereich N1 weist die Griffmulde P1 auf, welche zur Aussparung N0 hin offen ist. Der zweite Schenkelbereich N2 weist die Griffmulde P2 auf, welche zur Aussparung N0 hin offen ist.

Dadurch kann die Haltestruktur N sicherer gegriffen werden, um sie beispielsweise in die Mulde 28 einzusetzen oder aus der Mulde 28 herauszuheben. Wenn auf dem Boden der Mulde 28 beispielsweise Label angebracht sind, sind diese durch die Aussparung hindurch von oben sichtbar.

Beispielsweise kann vorgesehen sein, dass die Haltestruktur wenigstens eine weitere zweite geräteseitige Schnittstelle aufweist, welche zu der zweiten geräteseitigen Schnittstelle AC versetzt, beispielsweise näher an einem der beiden Schenkelbereiche als an dem anderen der beiden Schenkelbereiche, angeordnet ist. Beispielsweise kann die Haltestruktur drei weitere zweite geräteseitige Schnittstellen aufweisen, welche entlang des Scheitelbereichs nebeneinander angeordnet sind.

Die Lagerungsstruktur 8 kann dann quer zur Längsachse der Lagerungsstruktur 8 versetzt relativ zu der Haltestruktur N fixiert werden, beispielsweise um einen einfacheren Zugang zur Lagerungsstruktur 8 zu ermöglichen.

In der Fig. 11 ist die Haltestruktur N um 180 Grad relativ zu der in der Fig. 9 gezeigten Orientierung der Haltestruktur N gedreht. Durch die in der Fig. 11 gezeigte Orientierung der Schenkelbereiche N1, N2 relativ zu der Lagerungsstruktur 8 wirken diese einem Kippen der Lagerungsstruktur 8 entgegen, wenn die Haltestruktur N mit der daran fixierten Lagerungsstruktur 8 auf einer Tischplatte abgestellt ist. Wenn die Haltestruktur N in die Mulde 28 formschlüssig aufgenommen ist, wirkt ein Formschluss der Haltestruktur N mit den Rändern der Mulde 28 dem Kippen entgegen. Insbesondere kann dieser Formschluss derart ausgebildet sein, dass ein Entfernen der Haltestruktur N von dem Boden der Mulde 28 nur durch vertikales Anheben der Haltestruktur N relativ zu der Mulde 28 möglich ist.

Die zweite geräteseitige Schnittstelle AC weist das Schnittstellenelement AC1 und das Schnittstellenelement AC2 auf. Die tischseitige Schnittstelle BC weist das Schnittstellenelement BC1 und das Schnittstellenelement BC2 auf.

Die lagerungsseitige Schnittstelle C weist das Schnittstellenelement C2, das zu den Schnittstellenelementen AC2 und BC2 korrespondierend ausgebildet ist. Die lagerungsseitige Schnittstelle C weist ein weiteres Schnittstellenelement auf, das zu den Schnittstellenelementen AC1 und BC1 korrespondierend ausgebildet ist.

Die lagerungsseitige Schnittstelle C weist das Schnittstellenelement C3 in Form eines vorkragenden Absatzes auf. Die zweite geräteseitige Schnittstelle AC weist eine Kontaktfläche für einen Formschluss mit dem Schnittstellenelement C3 auf. Durch diesen Formschluss wird eine stabile Ausrichtung der Längsachse der Lagerungsstruktur 8 relativ zu der Haltestruktur N unterstützt. Die erste geräteseitige Schnittstelle 2C weist eine Kontaktfläche für einen Formschluss mit dem Schnittstellenelement C3 auf. Durch diesen Formschluss wird eine stabile Ausrichtung der Längsachse der Lagerungsstruktur 8 relativ zu der Gantry 20 unterstützt.

Die Gummipuffer A1 wirken einem Verrutschen der Haltestruktur N relativ zu der Tischplatte entgegen, wenn die Kontaktfläche der ersten Teilstruktur A der Haltestruktur N an der Tischplatte anliegt. Die Elemente B1 können beispielsweise Gummipuffer zur Verwendung an dem Boden der Mulde 28 und/oder Zugangsöffnungen für die Montage der Gummipuffer A1 sein.

## Patentansprüche

1. Lagerungsstruktur (8) zur Lagerung eines Untersuchungsobjekts (13) einer pädiatrischen Computertomographie-Untersuchung,
- wobei die Lagerungsstruktur (8) eine Auflagefläche (80) für das Untersuchungsobjekt (13), eine erste Seitenwand (81) und eine zweite Seitenwand (82) aufweist,
- wobei sich die Auflagefläche (80), die erste Seitenwand (81) und die zweite Seitenwand (82) jeweils entlang einer Längsachse der Lagerungsstruktur (8) flächig erstrecken,
- wobei die erste Seitenwand (81) und die zweite Seitenwand (82) derart relativ zu der Auflagefläche (80) aufragen, dass das Untersuchungsobjekt (13) zwischen der ersten Seitenwand (81) und der zweiten Seitenwand (82) auf die Auflagefläche (80) gelegt werden kann.

2. Lagerungsstruktur (8) nach Anspruch 1,
- wobei in der ersten Seitenwand (81) ein Langloch (L1) zum Einführen einer ersten Handfläche einer Bedienperson derart ausgebildet ist, dass die erste Handfläche einen an dieses Langloch (L1) grenzenden Randbereich der ersten Seitenwand (81) umschließen kann und/oder
- wobei in der zweiten Seitenwand (82) ein Langloch (L2) zum Einführen einer zweiten Handfläche einer Bedienperson derart ausgebildet ist, dass die zweite Handfläche einen an dieses Langloch (L2) grenzenden Randbereich der zweiten Seitenwand (82) umschließen kann.

3. Lagerungsstruktur (8) nach Anspruch 1 oder 2,
- wobei die Lagerungsstruktur (8) eine erste Verbindungseinheit (K1) und eine zweite Verbindungseinheit (K2) für eine Verbindung mit einem Haltegurt (H) aufweist,
- wobei die erste Verbindungseinheit (K1) derart an der ersten Seitenwand (81) befestigt ist und die zweite Verbindungseinheit (K2) derart an der zweiten Seitenwand (82) befestigt ist, dass die erste Seitenwand (81), der Haltegurt (H), die zweite Seitenwand (82) und die Auflagefläche (80) zusammen eine umlaufend geschlossene Kontur bilden, wenn der Haltegurt (H) mit der ersten Verbindungseinheit (K1) und mit der zweiten Verbindungseinheit (K2) verbunden ist.

4. Lagerungsstruktur (8) nach einem der Ansprüche 1 bis 3,
- wobei die Lagerungsstruktur (8) eine Rückwand (83) aufweist,
- wobei die Rückwand (83) relativ zu der Auflagefläche (80) aufragt und sich quer zu der Längsachse der Lagerungsstruktur (8) von der ersten Seitenwand (81) bis zu der zweiten Seitenwand (82) flächig erstreckt.

5. Lagerungsstruktur (8) nach Anspruch 4,
- wobei in der Rückwand (83) ein Loch (L3) zum Aufhängen der Lagerungsstruktur (8) ausgebildet ist.

6. Lagerungsstruktur (8) nach einem der Ansprüche 1 bis 5,
- wobei die Auflagefläche (80), die erste Seitenwand (81) und die zweite Seitenwand (82) einstückig und/oder schalenförmig ineinander übergehen.

7. Lagerungsstruktur (8) nach einem der Ansprüche 1 bis 6,
- wobei die Lagerungsstruktur (8) eine Längs-Markierung (MA) aufweist, welche auf der Auflagefläche (80) angeordnet ist und sich mittig zwischen der ersten Seitenwand (81) und der zweiten Seitenwand (82) entlang der Längsachse der Lagerungsstruktur (8) erstreckt.

8. Computertomographiegerät (1), aufweisend eine Gantry (20) mit einer Öffnung (9) und die Lagerungsstruktur (8) nach einem der Ansprüche 1 bis 7,
- wobei sich die Öffnung (9) derart tunnelförmig entlang einer Systemachse (SA) der Gantry (20) erstreckt, dass die Lagerungsstruktur (8) und das Untersuchungsobjekt (13) zusammen entlang der Systemachse (SA) in die Öffnung (9) einführbar sind, wenn die Längsachse der Lagerungsstruktur (8) parallel zu der Systemachse (SA) ist und das Untersuchungsobjekt (13) zwischen der ersten Seitenwand (81) und der zweiten Seitenwand (82) auf der Auflagefläche (80) liegt,
- wobei das Computertomographiegerät (1) eine erste geräteseitige Schnittstelle (1C, 2C) aufweist,
- wobei die Lagerungsstruktur (8) eine lagerungsseitige Schnittstelle (C) aufweist,
- wobei mittels der ersten geräteseitigen Schnittstelle (1C, 2C) und der lagerungsseitigen Schnittstelle (C) eine erste lösbare Verbindung hergestellt werden kann, welche die Lagerungsstruktur (8) derart relativ zu der Gantry (20) fixiert, dass die Längsachse der Lagerungsstruktur (8) parallel zu der Systemachse (SA) ist.

9. Computertomographiegerät (1) nach Anspruch 8,
- wobei die Gantry (20) einen ersten Gantryteil (21) und einen zweiten Gantryteil (22) aufweist,
- wobei der erste Gantryteil (21) ein Projektionsdatenakquisitionssystem (40) aufweist,
- wobei der zweite Gantryteil (22) die erste geräteseitige Schnittstelle (2C) aufweist,
- wobei der erste Gantryteil (21) relativ zu dem zweiten Gantryteil (22) derart bewegbar gelagert ist, dass eine Translationsbewegung des ersten Gantryteils (21) relativ zu dem zweiten Gantryteil (22) parallel zu der Systemachse (SA) ausgeführt werden kann, während gleichzeitig dazu die Lagerungsstruktur (8) relativ zu dem zweiten Gantryteil (22) ruht, wobei mittels der ersten geräteseitigen Schnittstelle (1C, 2C) und der lagerungsseitigen Schnittstelle (C) die erste lösbare Verbindung hergestellt ist, welche die Lagerungsstruktur (8) derart relativ zu der Gantry (20) fixiert, dass die Längsachse der Lagerungsstruktur (8) parallel zu der Systemachse (SA) ist.

10. Computertomographiegerät (1) nach Anspruch 9,
- wobei die Lagerungsstruktur (8) eine erste Quer-Markierung (MC) und eine zweite Quer-Markierung (MD) aufweist, welche jeweils auf der Auflagefläche (80) angeordnet sind und sich senkrecht zu der Längsachse der Lagerungsstruktur (8) erstrecken,
- wobei die erste Quer-Markierung (MC) und die zweite Quer-Markierung (MD) eine Erstreckung eines Untersuchungsbereichs, der durch die Translationsbewegung des ersten Gantryteils (21) relativ zu dem zweiten Gantryteil (22) für das Projektionsdatenakquisitionssystem (40) erreichbar ist, entlang der Systemachse (SA) markieren.

11. Computertomographiegerät (1) nach Anspruch 9 oder 10,
- wobei der zweite Gantryteil (22) eine zweite geräteseitige Schnittstelle (AC) aufweist,
- wobei mittels der zweiten geräteseitigen Schnittstelle (AC) und der lagerungsseitigen Schnittstelle (C) eine zweite lösbare Verbindung hergestellt werden kann, welche die Lagerungsstruktur (8) derart relativ zu der Gantry (20) fixiert, dass die Längsachse der Lagerungsstruktur (8) senkrecht und windschief zu der Systemachse (SA) ist.

12. Computertomographiegerät (1) nach Anspruch 11,
- wobei der zweite Gantryteil (22) ein Fahrwerk aufweist,
- wobei das Fahrwerk zum Bewegen der Gantry (20) relativ zu einem Boden eines Untersuchungsraumes parallel zu einer Translationsrichtung eingerichtet ist, wobei die Translationsrichtung senkrecht zu der Systemachse (SA) ist.

13. Computertomographiegerät (1) nach Anspruch 11 oder 12,
- wobei das Computertomographiegerät (1) eine Haltestruktur (N) für die Lagerungsstruktur (8) aufweist,
- wobei eine erste Teilstruktur (A) der Haltestruktur (N) die zweite geräteseitige Schnittstelle (AC) aufweist,
- wobei der zweite Gantryteil (22) eine Mulde (28) zur formschlüssigen Aufnahme der Haltestruktur (N) derart aufweist, dass die erste Teilstruktur (A) der Haltestruktur (N) aus der Mulde (28) herausragt, wenn die Haltestruktur (N) in die Mulde (28) formschlüssig aufgenommen ist.

14. Computertomographiegerät (1) nach Anspruch 13,
- wobei eine zweite Teilstruktur (B) der Haltestruktur (N) eine tischseitige Schnittstelle (BC) aufweist,
- wobei mittels der tischseitigen Schnittstelle (BC) und der lagerungsseitigen Schnittstelle (C) eine dritte lösbare Verbindung hergestellt werden kann, welche die Lagerungsstruktur (8) derart relativ zu der Haltestruktur (N) fixiert, dass die Längsachse der Lagerungsstruktur (8) im Wesentlichen parallel zu einer Tischplatte ist, wenn die Haltestruktur (N) derart auf der Tischplatte abgestellt ist, dass eine Kontaktfläche der ersten Teilstruktur (A) der Haltestruktur (N) an der Tischplatte anliegt.

15. Computertomographiegerät (1) nach Anspruch 13 oder 14,
- wobei die Haltestruktur (N) einen ersten Schenkelbereich (N1), einen zweiten Schenkelbereich (N2) und einen Scheitelbereich (N3) aufweist,
- wobei der Scheitelbereich (N3) die zweite geräteseitige Schnittstelle (AC) und/oder die tischseitige Schnittstelle (BC) aufweist und sich von dem ersten Schenkelbereich (N1) bis zu dem zweiten Schenkelbereich (N2) erstreckt,
- wobei sich der erste Schenkelbereich (N1) und der zweite Schenkelbereich (N2) parallel zu der Längsachse der Lagerungsstruktur (8) weg von dem Scheitelbereich (N3) erstrecken, wenn die zweite lösbare Verbindung oder die dritte lösbare Verbindung hergestellt ist.
